# FASCICULE DE BREVET EUROPEEN

(11) **EP 1 696 954 B1**
(45) Date de publication et mention de la délivrance du brevet: **29.10.2008**
(21) Numéro de dépôt: 04805451.4
(22) Date de dépôt: 15.11.2004
(51) Int. Cl.: A61K 39/39, A61K 39/21, A61K 39/245, A61K 39/215

(54) **COMPOSITION VACCINALE ADJUVANTÉE PAR UNE ALKYLPHOSPHATIDYLCHOLINE**
IMPFSTOFFZUSAMMENSETZUNG IM GEMISCH MIT EINEM ALKYLPHOSPHATIDYLCHOLIN
VACCINE COMPOSITION ADMIXED WITH AN ALKYLPHOSPHATIDYLCHOLINE

(30) Priorité: 17.11.2003 FR 0313406
(43) Date de publication de la demande: 06.09.2006
(73) Titulaire: Sanofi Pasteur, 69367 Lyon Cedex 07 (FR)
(72) Inventeur: HAENSLER, Jean, F-69290 Grezieu la Varenne (FR)
(74) Mandataire: Kerneis, Daniéle
(86) Numéro de dépôt international: PCT/FR2004/002911
(87) Numéro de publication internationale: WO 2005/049080

(56) Documents cités:
- WO-A-02/26209
- JIAO X ET AL: "Modulation of cellular immune response against hepatitis c virus nonstructural protein 3 by cationic liposome encapsulated DNA immunization." HEPATOLOGY, vol. 37, no. 2, février 2003 (2003-02), pages 452-460, XP008032301 ISSN: 0270-9139 cité dans la demande
- GORMAN C M ET AL: "Efficient in vivo delivery of DNA to pulmonary cells using the novel lipid EDMPC" GENE THERAPY, vol. 4, no. 9, 1997, pages 983-992, XP002286480 ISSN: 0969-7128
- MACDONALD R C ET AL: "Physical and biological properties of cationic triesters of phosphatidylcholine" BIOPHYSICAL JOURNAL, vol. 77, no. 5, novembre 1999 (1999-11), pages 2612-2629, XP002286481 ISSN: 0006-3495
- ROSENZWEIG H S ET AL: "O-alkyl dioleoylphosphatidylcholinium compounds: The effect of varying alkyl chain length on their physical properties and in vitro DNA transfection activity" BIOCONJUGATE CHEMISTRY, vol. 11, no. 3, mai 2000 (2000-05), pages 306-313, XP002286482 ISSN: 1043-1802

## Description

La présente invention est relative au domaine des vaccins et plus particulièrement à des vaccins adjuvés. En particulier, l'invention concerne des compositions pharmaceutiques comprenant au moins un antigène vaccinal et au moins un dérivé ester phosphorique de phosphatidylcholine.

Selon l'art antérieur, il est connu de vouloir augmenter ou orienter la réponse immunitaire induite par les antigènes présents dans un vaccin, au moyen d'adjuvants. Ceci peut être souhaitable car l'antigène, administré seul, n'est pas suffisamment immunogène, à cause notamment de son très haut degré de pureté, ou parce qu'on souhaite réduire la quantité d'antigènes présents dans le vaccin ou le nombre de rappels à effectuer, ou encore parce qu'on souhaite allonger la durée de protection conférée par le vaccin. Parfois, il s'agit de modifier qualitativement plutôt que quantitativement la réponse induite.

Bien que l'art antérieur abonde en propositions de produits pouvant être utilisés comme adjuvants, on remarque que la plupart des vaccins adjuvés faisant l'objet d'une autorisation de mise sur le marché en médecine humaine, sont adjuvés au moyen d'un sel d'aluminium, ou d'une émulsion.

Or, il existe une demande réelle de disposer de nouveaux adjuvants dont les qualités permettront de modifier la réponse immune tout en conservant les qualités d'une administration en toute sécurité.

Par ailleurs, dans un autre domaine qui est celui de la transfection, il a été proposé dans l'état de la technique, d'utiliser des lipides cationiques. Ainsi, par exemple, dans la publication intitulée « Modulation of Cellular Immune Response Against Hepatitis C Virus Nonstructural Protein 3 by Cationic Liposome Encapsulated DNA Immunization » (Jiao X et al., Hepatology (2003) 37(2): 452-460), plusieurs lipides cationiques constituant des liposomes sont testés pour leur capacité à véhiculer l'ADN plasmidique d'intérêt vers les cellules. Cette publication est d'un intérêt dans le domaine des vaccins car l'ADN transfecté code pour des antigènes contre lesquels on souhaite une réponse du système immunitaire. Pour que la réaction du système immunitaire soit bonne, il faut tout d'abord que l'antigène soit exprimé, et donc que l'ADN soit « délivré » dans les meilleures conditions aux cellules capables de l'exprimer. Le rôle joué par les agents de transfection qui servent de « véhicule » à l'ADN est différent du rôle joué par un adjuvant qui accompagne un antigène vaccinal présent dans une composition pharmaceutique. Les résultats obtenus par les auteurs de cette publication montrent que la réponse obtenue est plus importante lorsque l'ADN est transporté au moyen de liposomes plutôt que lorsque l'ADN est injecté « nu » ; cependant, suivant la nature des lipides utilisés pour former les liposomes, les réponses obtenues varient en nature et en intensité.

Malgré la littérature abondante sur les divers produits pouvant être utilisés comme adjuvants, il existe toujours un besoin de disposer d'un produit pouvant être utilisé sans risque pour l'organisme auquel il est administré, et qui permette d'accroître et/ou de modifier la réponse du système immunitaire vis-à-vis de l'antigène vaccinal avec lequel il est administré.

C'est un des buts de l'invention que de proposer un tel produit.

Un autre but de l'invention est de proposer un adjuvant vaccinal facilement disponible, dont le coût est tel qu'il puisse être ajouté aux compositions vaccinales sans en augmenter le prix de revient de façon prohibitive.

Pour atteindre ces buts, la présente invention a pour objet une composition pharmaceutique comprenant au moins un antigène vaccinal autre qu'un ADN codant, **caractérisée en ce qu'**elle comprend en outre au moins un dérivé ester phosphorique de phosphatidylcholine ayant la structure : 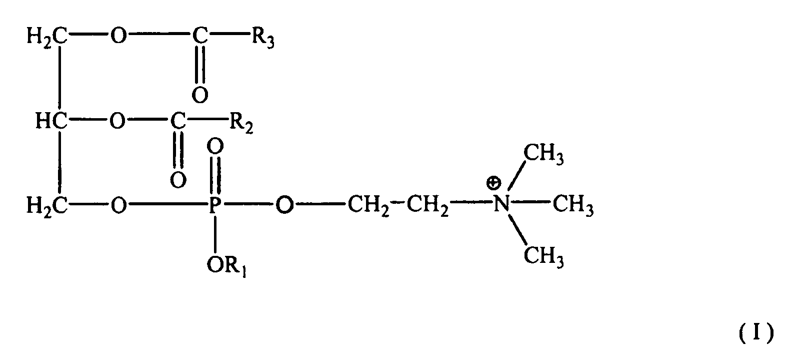 dans laquelle :
- R₁ est un alkyle inférieur, ayant au plus 5 atomes de carbone,
- R₂ et R₃ sont identiques ou différents, et peuvent chacun représenter des chaînes linéaires hydrocarbonées ayant de 13 à 21 atomes de carbone.

Selon un mode de réalisation particulier, R₁ représente le radical éthyl ; ainsi, la dégradation de ce dérivé dans l'organisme auquel il aura été administré produira des produits biocompatibles : les phosphatidylcholines et l'éthanol.

Selon un mode particulier de l'invention, R₂ et R₃ sont choisis parmi les radicaux provenant des acides gras suivants : acide myristique, acide palmitique, acide stéarique, acide oléique.

Selon un mode particulièrement avantageux, R₂ et R₃ sont tous 2 identiques et représentent le radical de l'acide oléique. Un tel produit pouvant être totalement préparé par synthèse chimique présente toutes les garanties de sécurité et de reproductibilité souhaitées pour un usage pharmaceutique.

L'invention a également pour objet l'utilisation d'un dérivé ester phosphorique de phosphatidylcholine ayant la structure : 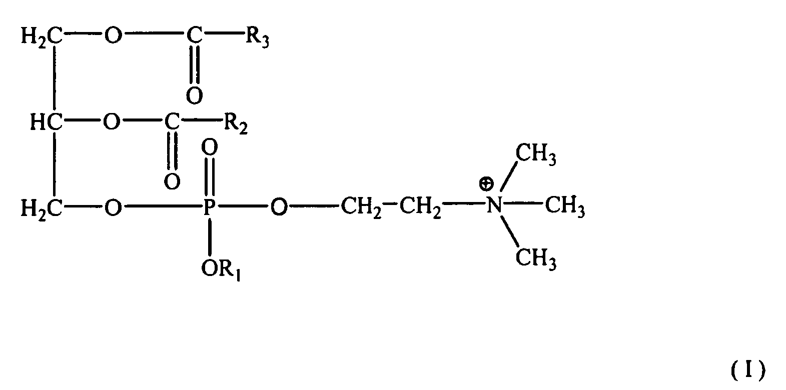 dans laquelle :
- R₁ est un alkyle inférieur, ayant au plus 5 atomes de carbone,
- R₂ et R₃ sont identiques ou différents, et peuvent chacun représenter des chaînes linéaires hydrocarbonées ayant de 13 à 21 atomes de carbone,
pour la préparation d'un adjuvant vaccinal.

De nombreux autres avantages de la présente invention apparaîtront à la lecture de la description détaillée qui va suivre.

La présente invention est relative à une composition pharmaceutique comprenant au moins un antigène vaccinal. Par antigène vaccinal, on entend un antigène susceptible d'induire une réponse du système immunitaire lorsqu'il est administré à l'homme ou à un animal. Cette réponse du système immunitaire peut se traduire par une production d'anticorps ou par une activation de certaines cellules, notamment les cellules présentatrices d'antigènes ( ex : les cellules dendritiques), les lymphocytes T, les lymphocytes B.

La composition pharmaceutique peut être une composition à visée prophylactique ou à visée thérapeutique, ou encore les deux.

Elle peut être administrée par toutes les voies habituellement utilisées ou préconisées pour les vaccins : voie parentérale, voie muqueuse, et se présenter sous diverses formes : liquide injectable ou pulvérisable, formulation lyophilisée ou séchée par atomisation ou à l'air,...etc. Elle peut être administrée au moyen d'une seringue ou au moyen d'un injecteur sans aiguille pour injection intramusculaire, sous-cutanée ou intradermique. Elle peut aussi être administrée grâce à un nébuliseur capable de délivrer une poudre sèche ou un spray liquide au niveau des muqueuses, qu'elles soient nasales, pulmonaires, vaginales ou rectales.

Les antigènes vaccinaux utilisés dans les compositions pharmaceutiques selon la présente invention sont des antigènes « directs », c'est-à-dire qu'il ne s'agit pas d'ADN codant pour ces antigènes, mais les antigènes eux-mêmes ; il peut s'agir d'un germe entier ou seulement d'une partie de ce germe ; ainsi parmi les antigènes utilisés habituellement dans les vaccins, on peut notamment citer :
- les polysaccharides, qu'ils soient seuls ou conjugués à des éléments porteurs, tels que les protéines porteuses,
- les germes entiers vivants atténués,
- les germes inactivés,
- les peptides et protéines recombinants,
- les glycoprotéines, les glycolipides, les lipopeptides,
- les peptides synthétiques,
- les germes éclatés dans le cas de vaccins appelés vaccins « splittés ».

Ces antigènes sont des antigènes utilisés ou susceptibles d'être utilisés pour le traitement ou la prévention de diverses maladies telles que par exemple : diphtérie, tétanos, polio, rage, coqueluche, hépatites A, B, C, fièvre jaune, fièvre typhoïde, varicelle, rougeole, oreillons, rubéole, encéphalite japonaise, méningites, infections à pneumocoques, infections à rotavirus, SIDA, cancers, tuberculose, maladie de Lyme, infections à RSV, herpès, affections bactériennes provoquées par Chlamydia, Neisseria gonorrheae, Streptococcus pneumoniae, Moraxella catarrhalis, Staphylococcus aureus ou Haemophilus influenza type B, la malaria, les leshmanioses, les listerioses, ...etc.

La composition pharmaceutique selon l'invention peut être une composition destinée à l'immunisation contre un seul pathogène ou cancer, c'est-à-dire qu'elle comprend un ou plusieurs antigènes d'un seul pathogène ou cancer, ou bien être une composition destinée à l'immunisation contre plusieurs pathogènes ou cancers (on parle alors de combinaison vaccinale).

L'action du dérivé ester phosphorique de la phosphatidylcholine utilisé est d'adjuver la composition vaccinale, c'est-à-dire d'accroître ou de modifier la réponse du système immunitaire de l'organisme auquel la composition vaccinale est administrée, par rapport à la réponse qui serait obtenue en absence d'un tel composé. En particulier, il peut s'agir d'une augmentation de la réponse humorale, ou de la réponse cellulaire, ou des deux. L'action peut également être, non pas une augmentation de la réponse, mais une orientation différente de la réponse induite : par exemple, orientation vers une réponse cellulaire plutôt qu'une réponse humorale, production de certaines cytokines plutôt que d'autres, production de certains types ou sous-types d'anticorps plutôt que d'autres, stimulation de certaines cellules plutôt que d'autres, etc... L'action de l'adjuvant peut également consister à augmenter la durée de la réponse immune dans le temps. Il peut aussi s'agir de permettre de diminuer le nombre d'administrations nécessaires pour obtenir une protection de l'individu immunisé, ou encore diminuer la quantité d'antigène contenue dans la dose administrée.

L'action adjuvante du dérivé selon l'invention peut être obtenue, soit lorsqu'il est associé à l'antigène ou aux antigènes de la composition pharmaceutique lors de leur administration, i.e. lorsqu'il est présent directement dans la composition pharmaceutique, ou encore lorsqu'il est administré séparément de l'antigène ou des antigènes dont on veut modifier le pouvoir immunogène. On préfère cependant l'utiliser dans la même composition pharmaceutique que l'antigène ou les antigènes à administrer.

Aux fins de la présente invention, on entend par phosphatidylcholine des phospholipides constitués d'une molécule de glycérol, de 2 acides gras, d'un groupe phosphate et de la choline. Ces composés encore appelés lécithines, varient en fonction de leurs acides gras.

Pour les besoins de l'invention, les 2 acides gras R₂ et R₃ peuvent être identiques ou différents, saturés ou insaturés ; on utilise de préférence des phosphatidylcholines provenant de l'estérification d'acides gras ayant au moins 13 atomes de carbone, et notamment des acides suivants : myristique, palmitique, stéarique, ou oléique.

Par radical alkyle inférieur R₁, on entend un radical alkyle ayant au plus 5 atomes de carbone. En effet, selon l'invention, le dérivé de phosphatidylcholine est un ester phosphorique d'une phosphatidylcholine ; il peut s'agir d'un ester provenant de la réaction de la phosphatidylcholine et de méthanol, d'éthanol, d'un propanol, d'un butanol ou d'un pentanol ; on a obtenu de particulièrement bons résultats avec un ester provenant de l'éthanol.

Les composés convenant aux fins de l'invention peuvent être obtenus par synthèse chimique complète, ou encore par estérification de phosphatidylcholines naturelles, telles que la phosphatidylcholine extraite du soja ou des oeufs. Ce sont des composés que l'on peut utiliser sous forme de sel pharmaceutiquement acceptable, et notamment de chlorure. Parmi les composés convenant particulièrement bien , on peut citer la 1,2-dimyristoyl-*sn*-glycero-3-éthylphosphocholine, la 1,2-dipalmitoyl-sn-glycero-3-éthylphosphocholine, la 1,2-distearoyl-sn-glycero-3-éthylphosphocholine, la 1,2-dioleoyl-*sn*-glycero-3-éthylphosphocholine ou encore la 1,2-palmitoyl-oleoyl-*sn*-glycero-3-éthylphosphocholine, disponibles notamment auprès de la Société Avanti® Polar Lipids Inc. Ces composés sont disponibles sous forme de poudre ou en solution dans du chloroforme.

Selon l'invention, les composés sont dispersés dans l'eau ou dans un tampon aqueux pour former une suspension qui est alors mélangée avec une solution comprenant les antigènes vaccinaux, ou bien la suspension contenant le dérivé ester phosphorique de phosphatidylcholine est utilisée pour reprendre un lyophilisat comprenant les antigènes vaccinaux. De façon alternative, il est possible de disperser ou d'hydrater le dérivé ester de phosphatidylcholine avec de l'eau ou un tampon comprenant déjà au moins un antigène vaccinal.

Les dérivés esters phosphoriques de phosphatidylcholine utilisés peuvent alors former des vésicules lipidiques, ou liposomes, dont la taille est avantageusement comprise entre 50 et 220nm.

Il est également possible d'utiliser les adjuvants selon l'invention dans une composition comprenant une émulsion.

Les exemples qui suivent illustrent, de façon non limitative, des exemples de réalisation de la présente invention.

### Exemple 1 : Compositions vaccinales ayant comme antigène de la protéine TAT.

### 1.1. Préparation des compositions

On prépare des compositions vaccinales comprenant à titre d'antigène vaccinal, une protéine recombinante susceptible d'être utilisée dans un vaccin contre le SIDA ; il s'agit de la protéine TAT III B détoxifiée qui est obtenue par expression chez E. coli et purification par différentes étapes de chromatographie, puis inactivation chimique, ainsi que cela est décrit dans la demande WO99/33346, où elle est identifiée sous le terme de Tat carboxyméthylée.

Les compositions sont préparées de la manière décrite ci-après.

On dispose de poudre de chlorure de 1,2 dioleoyl-sn-glycero-3 ethylphosphocholine (ethyl PC) fournie par la Société AVANTI® POLAR LIPIDS Inc. que l'on solubilise dans un mélange de chloroforme/méthanol 4/1 afin d'obtenir une concentration de 2 mg/mL.

3,18 mL de la solution obtenue, soit 6,36 mg d'éthyl PC sont introduits dans un ballon en verre ; le solvant est éliminé à l'aide d'un évaporateur rotatif jusqu'à obtention d'un film lipidique régulier sur les parois du ballon. Ce film est séché ensuite sous vide poussé pour éliminer toute trace de solvant résiduel.

Le film est ensuite réhydraté par 4 ml d'eau distillée à 40°C en utilisant un bain à sonication. La dispersion des vésicules ainsi obtenue est ensuite extrudée à travers plusieurs membranes de polycarbonate (0,8 µm ; 0,4 µm et 0,2 µm) montées sur un Extruder (Lipex Biomembranes, Inc.) thermostaté à 50°C sous pression d'azote.

La suspension liposomale alors obtenue, ayant une concentration de 1,59 mg/mL est mélangée volume à volume à la solution de Tat concentrée à 200 µg/mL en tampon TRIS 100 mmol/L, NaCl 200 mmol/L à pH 7,4.

Une composition comprenant uniquement l'antigène TAT, sans adjuvant est également préparée.

On obtient ainsi des doses de 200µl dont les compositions sont les suivantes :
1) 20µg de TAT
2) 20µg de TAT et 159µg d'éthyl PC.

### 1.2. Immunisation.

On dispose de 2 groupes de 6 souris BALB/c femelles âgées de 8 semaines, à qui l'on injecte une des compositions préparées au paragraphe 1.1, par voie sous-cutanée, à raison d'une dose de 200µl par souris ; les injections sont réalisées à J0 et à J21.

On prélève des échantillons sanguins au sinus retro-orbital à J14 pour l'appréciation de la réponse primaire et à J35 pour la réponse secondaire. La détermination du taux d'IgG1 et d'IgG2a spécifiques est effectuée grâce à des tests ELISA standardisés.

Les souris sont sacrifiées à J37 ; on prélève leur rate et on isole les splénocytes.

Les résultats obtenus en ce qui concerne les réponses humorales sont récapitulés dans le tableau ci-dessous, où les taux d'IgG sont exprimés en unités arbitraires ELISA (log10). Pour chaque groupe de souris, la valeur indiquée est le titre géométrique moyen des valeurs obtenues pour chacune des souris.

| Groupes de souris | Taux IgG1 à J14 | Taux IgG2a à J14 | Taux IgG1 à J35 | Taux IgG2a à J35 |
|---|---|---|---|---|
| Tat seule | 1,943 | 1,045 | 3,950 | 2,437 |
| Tat + EthylPC | 2,931 | 2,429 | 4,889 | 4,368 |

Les résultats obtenus montrent que les compositions selon l'invention permettent d'obtenir une réponse humorale nettement supérieure à celle obtenue dans le cas où l'antigène est administré seul.

On observe que l'effet adjuvant est nettement marqué vis-à-vis de la réponse en IgG2a, ce qui est une indication d'une réponse immune plutôt orientée vers une réponse de type Th1.

Pour apprécier l'effet des compositions pharmaceutiques selon l'invention sur la réponse cellulaire, on effectue des comptages des cellules spléniques capables de produire de l'interféron γ par un test ELISPOT. Ce test est réalisé à la fois sur des cellules fraîches et sur des cellules restimulées.

Pour la réalisation du test, on cultive les cellules spléniques dans des plaques de culture cellulaire, à raison de 200 000 cellules par puits, en présence soit du milieu seul, soit de l'antigène TAT recombinante. Après 16 heures de culture, on révèle l'ELISPOT, i.e. que l'on compte le nombre de spots correspondant aux cellules sécrétant de l'interféron γ. Les résultats obtenus sont résumés dans les tableaux ci-après ; les valeurs indiquées sont les valeurs moyennes (par groupe de souris), des différences calculées pour chaque souris entre le nombre de spots comptés par million de cellules dans les puits ayant de la TAT recombinante et le nombre de spots comptés par million de cellules dans les puits n'ayant que du milieu.

Le tableau ci-après résume les résultats obtenus sur cellules fraîches.

| Composition pharmaceutique testée | Nombre de spots par millions de cellules |
|---|---|
| TAT à 20 µg | 31,67 |
| TAT à 20 µg + EthylPC | 129,17 |

Le tableau ci-après résume les résultats obtenus sur cellules restimulées par de la TAT recombinante en présence d'IL2.

| Composition pharmaceutique testée | Nombre de spots par millions de cellules |
|---|---|
| TAT à 20 µg | 44,16 |
| TAT à 20 µg + EthylPC | 411,66 |

Ces résultats montrent l'effet positif obtenu en utilisant une composition pharmaceutique selon la présente invention, sur la stimulation des cellules CD4+.

### 2. Compositions vaccinales ayant comme antigène de la glycoprotéine gB du Cytomegalovirus.

### 2.1. Préparation des compositions.

On prépare des compositions vaccinales comprenant à titre d'antigène vaccinal une protéine recombinante dérivée d'une glycoprotéine d'enveloppe de la souche Towne du Cytomegalovirus (CMV) dénommée gB dont les séquences nucléotidique et protéique sont décrites dans le brevet USP 5,834,307. Cette protéine recombinante est produite par une lignée CHO recombinante transfectée par un plasmide dénommé pPRgB27clv4 qui contient un gène modifié de la gB. En effet, pour faciliter la production de cette protéine recombinante par la lignée CHO le gène de la gB a été modifié au préalable en supprimant la partie du gène qui code pour la région transmembranaire de la protéine gB correspondant à la séquence d'acides aminés comprise entre la Valine 677 et l'Arginine 752 et en introduisant 3 mutations ponctuelles de sorte que le site de clivage existant dans la gB native a été supprimée. En définitive la protéine recombinante produite par la lignée CHO recombinante correspond à une protéine gB tronquée dépourvue de site de clivage et de région transmembranaire dénommée gBdTM.

La construction du plasmide pPRgB27clv4 et la production de la protéine gB tronquée (gBdTM) par la lignée CHO recombinante sont décrites dans US 6,100,064. La purification de la protéine gB tronquée est réalisée sur colonne chromatographique d'immunoaffinité en utilisant l'anticorps monoclonal 15D8 décrit par Rasmussen L et al. (J. Virol. (1985) 55 : 274-280).

On dispose de poudre de chlorure de 1,2-distéaroyl-*sn*-glycero-3-éthylphosphocholine (Ethyl DSPC) et de chlorure de 1,2-dioleoyl-*sn*-glycero-3-éthylphosphocholine (Ethyl DOPC) fournis par la Société AVANTI® POLAR LIPIDS Inc. Pour chacun des produits, on effectue les opérations suivantes :

On solubilise 5mg de poudre dans 5ml de chloroforme/méthanol 4 :1 (vol/vol). La solution est séchée dans un ballon en verre à l'aide d'un évaporateur rotatif de manière à laisser un film lipidique sur les parois du ballon. Ce film est encore séché sous vide poussé pour éliminer toute trace de solvant résiduel puis repris dans 2,5ml d'eau à 60°C pour une concentration finale de 2mg/ml. La suspension liposomale résultante est homogénéisée par agitation au vortex 10 minutes, sonication dans un bain à ultrasons 5 minutes puis extrudée , à l'aide d'un extrudeur Lipex (Northern Lipids Inc.,Vancouver, CA) thermostaté à 50°C, en cinq passages à travers une membrane de polycarbonate de porosité 0,2 µm.

On prépare également une formulation comprenant de l'éthyl PC en émulsion.

Pour cela, on solubilise dans un ballon en verre 25 mg de chlorure de 1,2-dioléoyl-*sn-*glycero-3-éthylphosphocholine (Ethyl DOPC - Avanti Polar Lipids) à l'aide de 10 ml de chloroforme/méthanol 4 :1 (vol/vol). La solution obtenue est séchée à l'aide d'un évaporateur rotatif de manière à laisser un film lipidique sur les parois du ballon. Ce film est encore séché sous vide poussé pour éliminer toute trace de solvant résiduel.

Dans un bécher, on pèse 375 mg de Tween^{®} 80 végétal fourni par la Société Merck et on y ajoute 29,29 g d'eau, on agite jusqu'à complète dissolution du tensio-actif.

Puis, on reprend le film lipidique obtenu à l'étape précédente par cette solution de Tween^{®} 80. A cette suspension, on ajoute1475 mg de squalène (Fluka), et on homogénéise l'émulsion obtenue à l'Ultraturrax (8000-9500 rpm) puis au Microfluidiseur M110 (Microfluidics, Newton MA.), 20 passages à 60 psi.

La concentration finale en squalène de cette émulsion est de 5%.

On prépare les doses d'immunisation en mélangeant les formulations adjuvantes décrites ci-dessus ou de l'eau, à une solution stock aqueuse d'antigène à 160µg/ml de protéine gB, et à du tampon PBS, afin d'obtenir des doses de 5µl ayant les compositions suivantes :
1) 2µg de gB
2) 2µg de gB et 50µg d'éthyl DOPC,
3) 2µg de gB et 50µg d'éthyl DSPC,
4) 2µg de gB, 50µg d'éthyl DOPC, 1,25mg de squalène et 0,3mg de Tween^{®} 80.

### 2.2. Immunisation.

On dispose de 4 groupes de 8 souris non-consanguines OF1 femelles âgées de 8 semaines, à qui on injecte une des compositions préparées au paragraphe 2.1, par voie sous-cutanée, à raison d'une dose de 50µl par souris ; les injections sont réalisées à J0 et à J21.

On prélève des échantillons sanguins au sinus retro-orbital à J20 pour l'appréciation de la réponse primaire et à J34 pour la réponse secondaire. La détermination du taux d'IgGI et d'IgG2a spécifiques est effectuée grâce à des tests ELISA standardisés.

Les souris sont sacrifiées à J37 ; on prélève leur rate et on isole les splénocytes, que l'on stimule ou non avec de la protéine gB recombinante.

On effectue alors une détermination par tests ELISA de la concentration en cytokines (IL5, IFN-γ et TNF-α) dans les surnageants de cellules spléniques stimulées pendant 5 jours avec de la protéine gB, ainsi que dans les surnageants de cellules non stimulées afin d'en déduire par comparaison la production spécifique de cytokines.

Les résultats obtenus en ce qui concerne les réponses humorales sont récapitulés dans le tableau ci-dessous, où les taux d'IgG sont exprimés en unités arbitraires ELISA (log10). Pour chaque groupe de souris, la valeur indiquée est le titre géométrique moyen des valeurs obtenues pour chacune des souris.

| Groupes de souris | Taux IgG1 à J21 | Taux IgG2a à J21 | Taux IgG1 à J35 | Taux IgG2a à J35 |
|---|---|---|---|---|
| gB seule | 2,703 ± 0,773 | 1,624 ± 0,572 | 4,409 ± 0,564 | 3,187 ± 0,595 |
| gB + Ethyl DOPC | 3,673 ± 0,521 | 3,763 ± 0,371 | 4,472 ± 0,453 | 4,741 ± 0,260 |
| gB + Ethyl DSPC | 3,708 ± 0,545 | 3,869 ± 0,303 | 4,388 ± 0,393 | 4,600 ± 0,246 |
| gB+ Ethyl DSPC + émulsion | 4,205 ± 0,388 | 2,944 ± 0,445 | 5,294 ± 0,416 | 4,272 ± 0,348 |

Les résultats pour les cytokines sont reproduits dans le tableau ci-après ; les valeurs indiquées sont pour chaque cytokine, la différence en pg/ml entre la moyenne des quantités de cytokines mesurées pour les cellules restimulées par de la protéine gB, et la moyenne des quantités de cytokines mesurées pour les cellules mises en culture dans du milieu seul (considéré comme le bruit de fond du test) ; on peut donc considérer que les quantités indiquées sont les quantités produites spécifiquement en réponse à la stimulation par la protéine gB.

| Groupes de souris | IFN-γ | IL5 | TNF-α |
|---|---|---|---|
| gB seule | 2193 | 399 | 61 |
| gB + Ethyl DOPC | 56338 | 416 | 310 |
| gB+ Ethyl DSPC | 28756 | 32 | 93 |
| gB+ Ethyl DSPC + émulsion | 3432 | 4054 | 134 |

L'ensemble des résultats produits dans ce test montre le bon effet adjuvant des esters phosphoriques de phosphatidylcholine qui ont une grande capacité à augmenter la réponse immune de type Th1 (augmentation des IgG2a et de l'Interféron γ), sans pour autant inhiber la réponse de type Th2 déjà induite par l'antigène.

### 3. Compositions vaccinales administrées par voie intradermique.

### 3.1. Préparation des compositions.

On dispose de poudre de chlorure de 1,2-dioleoyl-*sn*-glycero-3-éthylphosphocholine (Ethyl DOPC) fournis par la Société AVANT® POLAR LIPIDS Inc dont on solubilise 5mg dans 10 ml de chloroforme/méthanol 4 :1 (vol/vol). La solution obtenue est séchée dans un ballon en verre à l'aide d'un évaporateur rotatif de manière à laisser un film lipidique sur les parois du ballon. Ce film est encore séché sous vide poussé pour éliminer toute trace de solvant résiduel puis repris dans 2.5 ml d'eau à 60°C pour une concentration finale de 2 mg/ml. La suspension liposomale résultante est homogénéisée par agitation au vortex 10 minutes, sonication dans un bain à ultrasons 5 minutes puis extrudée , à l'aide d'un extrudeur Lipex (Northern Lipids inc,Vancouver, CA) thermostaté à 50°C, en un passage sur membrane de polycarbonate de 0,8 µm puis un passage sur membrane de polycarbonate de 0,4 µm et enfin cinq passages à travers une membrane de polycarbonate de porosité 0.2 µm.

On dispose également d'une solution stock d'antigène constitué par de la protéine gB obtenue de la manière décrite à l'exemple précédent, à une concentration de 160µg/ml.

On mélange la solution d'antigène à la suspension d'adjuvant à 2 mg/ml dans des proportions calculées pour obtenir les vaccins expérimentaux ayant par dose de 50µl, les compositions suivantes :
1) 2µg de gB,
2) 2µg de gB et 50µg d'éthyl DOPC
3) 0,2µg de gB,
4) 0,2µg de gB et 50µg d'éthyl DOPC.

### 3.2 Immunisation

On dispose de 4 groupes de 8 souris OF1 âgées de 8 semaines.

Chaque groupe de souris reçoit l'une des 4 formulations indiquées ci-dessus, par voie intradermique.

Les immunisations sont réalisées à J0 et à J20.

On prélève des échantillons sanguins au sinus retro-orbital à J20 avant la 2^{nde} immunisation pour l'appréciation de la réponse primaire et à J35 5 pour la réponse secondaire

La détermination du taux d'IgG1 et d'IgG2a spécifiques est effectuée grâce à des tests ELISA standardisés.

Les souris sont sacrifiées à J35 ; on prélève leur rate et on isole les splénocytes, que l'on stimule ou non avec de la protéine gB recombinante.

On effectue alors une détermination par tests ELISA de la concentration en cytokines (IL5, IFN-γ et TNF-α) dans les surnageants de cellules spléniques stimulées pendant 5 jours avec de la protéine gB, ainsi que dans les surnageants de cellules non stimulées afin d'en déduire par comparaison la production spécifique de cytokine.

Les résultats obtenus en ce qui concerne les réponses humorales sont récapitulés dans le tableau ci-dessous, où les taux d'IgG sont exprimés en unités arbitraires ELISA (log10). Pour chaque groupe de souris, la valeur indiquée est le titre géométrique moyen des valeurs obtenues pour chacune des souris

| Groupes de souris | Taux IgG1 à J20 | Taux IgG2a à J20 | Taux IgG1 à J35 | Taux IgG2a à J35 |
|---|---|---|---|---|
| 2µg gB | 2,095 | 1,097 | 4,478 | 2,829 |
| 2µg gB+ Ethyl DOPC | 4,111 | 3,574 | 5,023 | 4,766 |
| 0,2µg gB | 1,351 | 1,185 | 2,738 | 1,647 |
| 0,2µg gB+ Ethyl DOPC | 3,621 | 2,607 | 4,698 | 4,087 |

Les résultats pour les cytokines sont reproduits dans le tableau ci-après ; les valeurs indiquées sont pour chaque cytokine, la différence en pg/ml entre la moyenne des quantités de cytokines mesurées pour les cellules restimulées par de la protéine gB, et la moyenne des quantités de cytokines mesurées pour les cellules mises en culture dans du milieu seul (considéré comme le bruit de fond du test) ; on peut donc considérer que les quantités indiquées sont les quantités produites spécifiquement en réponse à la stimulation par la protéine gB.

| Groupes de souris | IFN-γ | IL5 | TNF-α |
|---|---|---|---|
| 2µg gB | 3295 | 1891 | 65 |
| 2µg gB + Ethyl DOPC | 49716 | 1373 | 638 |
| 0,2µg gB | 2892 | 102 | 135 |
| 0,2µg gB + Ethyl DOPC | 37877 | 690 | 533 |

Dans ce test, on voit que l'adjuvant selon l'invention est efficace par voie intradermique, et qu'il permet ou bien d'augmenter la réponse immunitaire pour une même quantité d'antigènes, ou de réduire la quantité d'antigènes présentes dans la dose injectée.

Ici, l'effet adjuvant est visible à la fois vis-à-vis de la réponse de type Th2 (IgG1) mais aussi, et même de façon très spectaculaire, vis-à-vis de la réponse de type Th1 (IgG2a, Interféron y).

### 4.Compositions vaccinales ayant un antigène du SARS.

### 4.1. Préparation des compositions.

On prépare une suspension virale d'un coronavirus humain inactivé, en l'occurrence le virus du SARS (Severe Acute Respiratory Syndrom), obtenu par culture sur lignée cellulaire puis inactivation ; la suspension utilisée est à une concentration de 7,56 log CCID50 avant inactivation ; elle est diluée au 1/10 afin d'obtenir une suspension ayant une concentration de 6,56 log CCID50.

On dispose de poudre de chlorure de 1,2-dioleoyl-*sn*-glycero-3-éthylphosphocholine (Ethyl DOPC) fournis par la Société AVANTI® POLAR LIPIDS Inc dont on solubilise 30 mg dans 10 ml de chloroforme/méthanol 4:1 (vol/vol). La solution obtenue est séchée dans un ballon en verre à l'aide d'un évaporateur rotatif de manière à laisser un film lipidique sur les parois du ballon. Ce film est encore séché sous vide poussé pour éliminer toute trace de solvant résiduel puis repris dans 5 ml d'eau à 60°C pour une concentration finale de 6 mg/ml. La suspension liposomale résultante est homogénéisée par agitation au vortex 10 minutes, sonication dans un bain à ultrasons 5 minutes puis extrudée, à l'aide d'un extrudeur Lipex (Northern Lipids Inc.,Vancouver, CA) thermostaté à 50°C, en cinq passages à travers une membrane de polycarbonate de porosité 0,2 µm.

On prépare des doses d'immunisation de 200µl, en effectuant les mélanges indiqués dans le tableau ci-après :

| Eau | Suspension virale à 6,56 log TCID₅₀ | Adjuvant | Tampon Phosphate | Quantité virus par dose |
|---|---|---|---|---|
| 1,418 ml | 110 µl dil. 1/10 | - | 472 µl | 10^{4,6}CCID₅₀ |
| 1,151 ml | 110 µl dil. 1/10 | 267 µl Ethyl DOPC | 472 µl | 10^{4,6}CCID₅₀ |
| 1,418 ml | 110 µl dil. 1 | - | 472 µl | 10^{5,6}CCID₅₀ |
| 1,151 ml | 110 µl dil. 1 | 267 µl Ethyl DOPC | 472 µl | 10^{5,6}CCID₅₀ |

### 4.2. Immunisation

On dispose de 4 groupes de 8 souris BALB/c que l'on immunise à 3 semaines d'intervalle avec l'une des formulations indiquées ci-dessus ; les injections sont réalisées en sous-cutané; les doses injectées sont à chaque fois de 200µl.

Des échantillons sanguins sont prélevés 2 semaines après chaque injection pour l'évaluation des réponses anticorps anti-virus SARS entier (inactivé) par dosage ELISA, soit à J14 pour la réponse primaire, et à J33 pour la réponse secondaire.

Les cellules spléniques sont prélevées à J33.

On évalue la réponse cellulaire spécifique du SARS, grâce à un dosage ELISPOT des cellules sécrétant de l'Interféron-γ, soit ex vivo, soit après stimulation in vitro avec du virus entier inactivé pendant 7 jours. Dans chacun des cas, les cellules spléniques (*ex vivo* ou restimulées *in* vitro) sont incubées 16 heures, soit avec du virus entier inactivé, soit avec des peptides 18-mères poolés dont les séquences sont chevauchantes (sur 10 acides aminés), et correspondent à différents antigènes du virus du SARS .

En outre, on apprécie la polarisation de la réponse cellulaire T helper induite, grâce à un dosage ELISA des cytokines sécrétées par les cellules spléniques stimulées par le virus inactivé entier ou par un pool de peptides.

Les résultats obtenus en ce qui concerne la réponse humorale sont regroupés dans le tableau ci-après ; ils sont exprimés en log10 d'unités arbitraires du test ELISA, et représentent les moyennes des titres géométriques de chaque groupe de souris.

| Composition immunisante | Taux IgG à J14 | Taux IgG à J33 |
|---|---|---|
| 10^{4,6}CCID₅₀ | 1 | 1,191 |
| 10^{4,6}CCID₅₀+ Ethyl DOPC | 1,246 | 2,424 |
| 10^{1,6}CCID₅₀ | 1,776 | 3,122 |
| 10^{5,6}CCID₅₀ + Ethyl DOPC | 2,621 | 4,387 |

Ces résultats montrent que l'adjuvant selon l'invention permet d'accroître la réponse humorale contre un antigène viral.

En ce qui concerne la réponse cellulaire, les résultats obtenus lors du comptage ELISPOT réalisé sur les cellules fraîches (*ex vivo*), stimulées par du virus entier sont représentés dans le tableau ci-après, qui indique la moyenne géométrique pour chaque groupe de souris.

| Composition immunisante | Nombre de spots/ 10⁶ cellules spléniques |
|---|---|
| 10^{4,6}CCID₅₀ | 2 |
| 10^{4,6}CCID₅₀ + Ethyl DOPC | 18 |
| 10^{5,6}CCID₅₀ | 17 |
| 10^{5,6}CCID₅₀ + Ethyl DOPC | 132 |

En ce qui concerne les résultats obtenus en réponse aux pools de peptides, les réponses varient selon les pools de peptides utilisés.

Les résultats en ELISPOT obtenus après restimulation avec du virus SARS inactivé ont également montré que la production d'interféron γ était augmentée en présence d'éthyl DOPC, en réponse à certains des peptides utilisés.

Ces résultats montrent que l'adjuvant selon l'invention permet d'accroître la réponse cellulaire T CD4+ contre un antigène viral.

En ce qui concerne les dosages des cytokines IFN-γ (cytokine Th1) et IL-5 (cytokine Th2) par ELISA, on a remarqué qu'après stimulation par du virus inactivé, la production d'IFN-γ était très augmentée pour les souris ayant reçu en plus de l'antigène de l'éthyl DOPC ; par contre, la production d'IL-5 n'était pas nettement modifiée.

Les résultats obtenus sont indiqués dans le tableau ci-après ; ils sont exprimés en pg/ml, et représentent les moyennes géométriques des groupes de souris.

| | Stimulation avec le virus entier inactivé | |
|---|---|---|
| Composition immunisante | IFN-γ (pg/ml) | IL-5 (pg/ml) |
| 10^{4,6}TCID₅₀ | 4910 | 2793 |
| 10^{4,6}TCID₅₀ + Ethyl DOPC | 9262 | 3037 |
| 10^{5,6}TCID₅₀ | 7223 | 3649 |
| 10^{5,6}TCID₅₀ + Ethyl DOPC | 51453 | 5270 |

Ces résultats montrent que l'adjuvant selon l'invention permet d'accroître la réponse cellulaire de type Th1.

Ainsi, l'ensemble de ces résultats montre que l'adjuvant selon l'invention permet d'accroître significativement la réponse immune induite lors de l'administration d'un antigène constitué par un virus inactivé, à la fois cellulaire (Th1) et humorale.

## Revendications

1. Composition pharmaceutique comprenant au moins un antigène vaccinal autre qu'un ADN codant, **caractérisée en ce qu'**elle comprend en outre au moins un dérivé ester phosphorique de phosphatidylcholine ayant la structure : 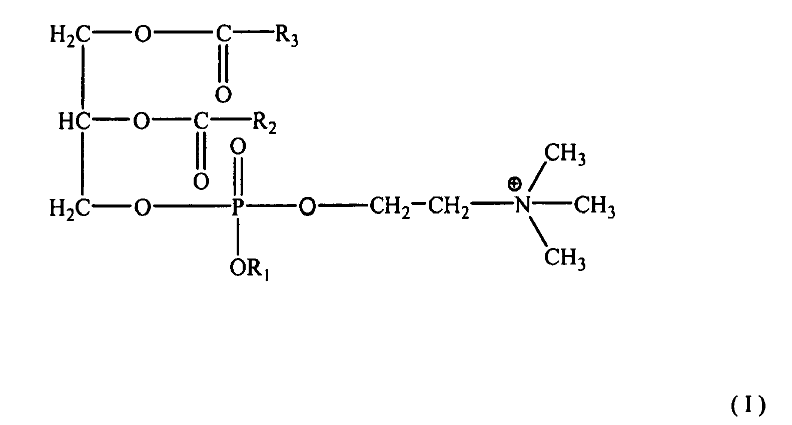 dans laquelle :
- R₁ est un alkyle inférieur, ayant au plus 5 atomes de carbone,
- R₂ et R₃ sont identiques ou différents, et peuvent chacun représenter des chaînes linéaires hydrocarbonées ayant de 13 à 21 atomes de carbone.

2. Composition selon la revendication précédente, **caractérisée en ce que** R₁ représente le radical éthyl.

3. Composition selon une des revendications précédentes, **caractérisée en ce que** R₂ et R₃ sont choisis parmi les radicaux provenant des acides gras suivants : acide myristique, acide palmitique, acide stéarique, acide oléique.

4. Composition selon une des revendications précédentes, **caractérisée en ce que** R₂ et R₃ sont tous deux identiques et représentent le radical de l'acide oléique.

5. Composition selon une des revendications précédentes, **caractérisée en ce qu'**elle comprend en outre une émulsion.

6. Utilisation d'un dérivé de phosphatidylcholine ayant la structure : 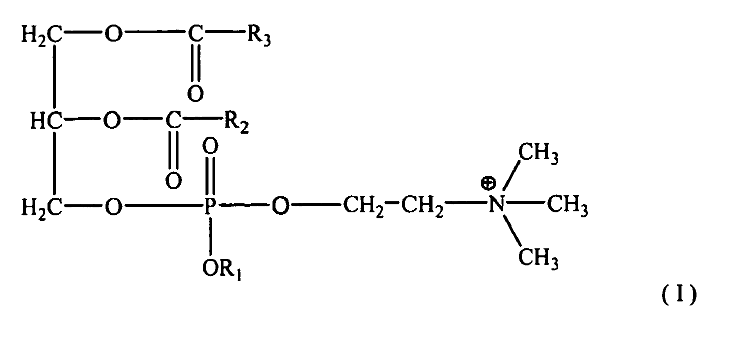 dans laquelle :
- R₁ est un alkyle inférieur, ayant au plus 5 atomes de carbone,
- R₂ et R₃ sont identiques ou différents, et peuvent chacun représenter des chaînes linéaires hydrocarbonées ayant de 13 à 21 atomes de carbone,
pour la préparation d'un adjuvant vaccinal.

7. Utilisation selon la revendication 6, **caractérisée en ce que** R₁ représente le radical éthyl.

8. Utilisation selon une des revendications 6 ou 7, **caractérisée en ce que** R₂ et R₃ sont choisis parmi les radicaux provenant des acides gras suivants : acide myristique, acide palmitique, acide stéarique, acide oléique.

9. Utilisation selon une des revendications 6 à 8, **caractérisée en ce que** R₂ et R₃ sont tous 2 identiques et représentent chacun le radical de l'acide oléique.

## Claims

1. Pharmaceutical composition comprising at least one vaccine antigen other than a coding DNA, **characterized in that** it also comprises at least one phosphoric ester derivative of phosphatidylcholine having the structure: 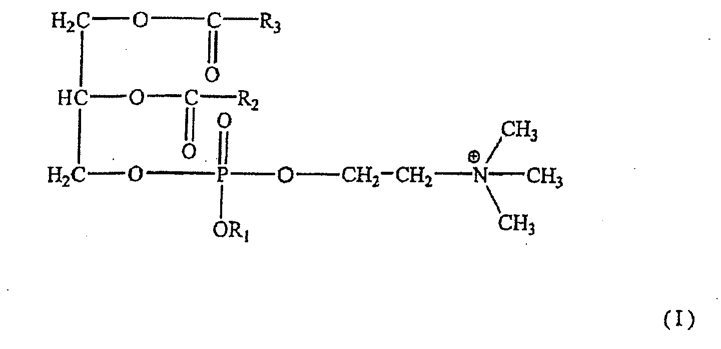 in which:
- R₁ is a lower alkyl having at most 5 carbon atoms,
- R₂ and R₃ are identical or different, and can each represent linear hydrocarbon-based chains having from 13 to 21 carbon atoms.

2. Composition according to the preceding claim, **characterized in that** R₁ represents the ethyl radical.

3. Composition according to any of the preceding claims, **characterized in that** R₂ and R₃ are chosen from the radicals originating from the following fatty acids: myristic acid, palmitic acid, stearic acid and oleic acid.

4. Composition according to one of the preceding claims, **characterized in that** R₂ and R₃ are both identical and represent the oleic acid radical.

5. Composition according to one of the preceding claims, **characterized in that** it also comprises an emulsion.

6. Use of a derivative of phosphatidylcholine having the structure: 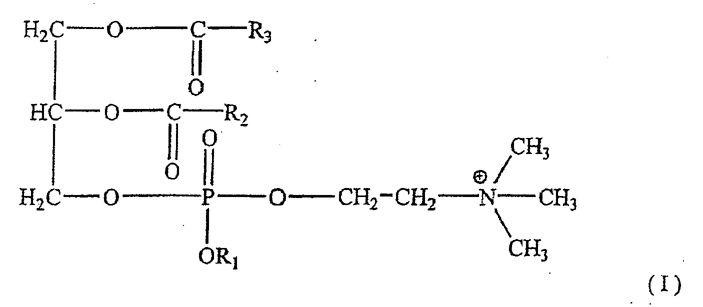 in which:
- R₁ is a lower alkyl having at most 5 carbon atoms,
- R₂ and R₃ are identical or different, and can each represent linear hydrocarbon-based chains having from 13 to 21 carbon atoms,
for preparing a vaccine adjuvant.

7. Use according to Claim 6, **characterized in that** R₁ represents the ethyl radical.

8. Use according to either of Claims 6 and 7, **characterized in that** R₂ and R₃ are chosen from the radicals originating from the following fatty acids: myristic acid, palmitic acid, stearic acid and oleic acid.

9. Use according to one of Claims 6 to 8, **characterized in that** R₂ and R₃ are both identical and each represent the oleic acid radical.

## Patentansprüche

1. Pharmazeutische Zusammensetzung, umfassend mindestens ein Impf-Antigen, das anders ist als eine codierende DNA, **dadurch gekennzeichnet, dass** sie außerdem mindestens ein Phosphorsäureester-Derivat von Phosphatidvlcholin mit der Struktur 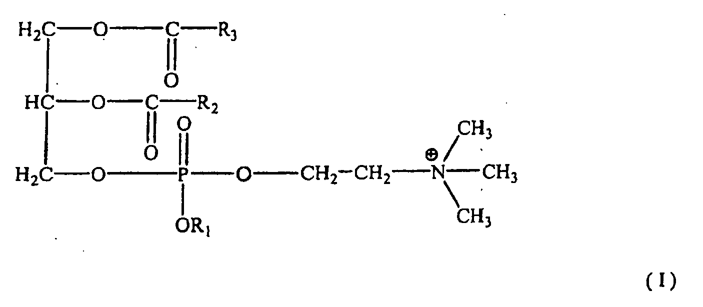 umfasst, in der
- R₁ ein niederes Alkyl mit höchstens 5 Kohlenstoffatomen darstellt,
- R₂ und R₃ gleich oder verschieden sind und jeweils lineare Kohlenwasserstoff-Ketten mit 13 bis 21 Kohlenstoffatomen darstellen können.

2. Zusammensetzung nach dem vorstehenden Anspruch, **dadurch gekennzeichnet, dass** R₁ den Rest Ethyl darstellt.

3. Zusammensetzung nach einem der vorstehenden Ansprüche, **dadurch gekennzeichnet, dass** R₂ und R₃ ausgewählt werden unter den Resten, die von den folgenden Fettsäuren stammen: Myristinsäure, Palmitinsäure, Stearinsäure, Oleinsäure.

4. Zusammensetzung nach einem der vorstehenden Ansprüche, **dadurch gekennzeichnet, dass** R₂ und R₃ beide gleich sind und den Rest der Oleinsäure darstellen.

5. Zusammensetzung nach einem der vorstehenden Ansprüche, **dadurch gekennzeichnet, dass** sie außerdem eine Emulsion umfasst.

6. Verwendung eines Phosphatidylcholin-Derivates mit der Struktur 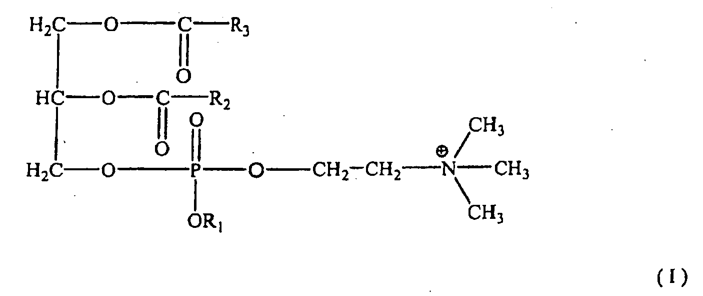 in der
- R₁ ein niederes Alkyl mit höchstens 5 Kohlenstoffatomen darstellt,
- R₂ und R₃ gleich oder verschieden sind und jeweils lineare Kohlenwasserstoff-Ketten mit 13 bis 21 Kohlenstoffatomen darstellen können,
für die Herstellung eines Impf-Adjuvans.

7. Verwendung nach Anspruch 6, **dadurch gekennzeichnet, dass** R₁ den Rest Ethyl darstellt.

8. Verwendung nach einem der Ansprüche 6 oder 7, **dadurch gekennzeichnet, dass** R₂ und R₃ ausgewählt werden unter den Resten, die von den folgenden Fettsäuren stammen: Myristinsäure, Palmitinsäure, Stearinsäure, Oleinsäure.

9. Verwendung nach einem der Ansprüche 6 bis 8, **dadurch gekennzeichnet, dass** R₂ und R₃ beide gleich sind und jeweils den Rest der Oleinsäure darstellen.
